(19) Europäisches Patentamt
European Patent Office
Office européen des brevets

(11) EP 3 760 116 A1

(12) EUROPEAN PATENT APPLICATION

(43) Date of publication:
06.01.2021 Bulletin 2021/01

(51) Int Cl.:
A61B 5/053 (2021.01)　　　A61B 5/11 (2006.01)
A61B 5/00 (2006.01)　　　G02C 11/00 (2006.01)
A61B 3/00 (2006.01)

(21) Application number: 19184156.8

(22) Date of filing: 03.07.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(71) Applicant: Vivior AG
8005 Zürich (CH)

(72) Inventors:
• Zakharov, Pavel
  8604 Volketswil (CH)

• Mrochen, Michael
  8183 Eglisau (CH)
• Moser, Urs
  8006 Zürich (CH)
• Weitnauer, Adrian
  8752 Näfels (CH)

(74) Representative: Frenkel, Matthias Alexander
Wuesthoff & Wuesthoff
Patentanwälte PartG mbB
Schweigerstrasse 2
81541 München (DE)

(54) **EYE BLINK SENSOR AND METHOD OF EXAMINING BLINKING OF AN EYE OF A USER**

(57) Disclosed is an eye blink sensor (1) comprising a detection system configured to detect an electrical field in front of an eye (4) of a user, wherein the detection system comprises a first electrode (2) configured to be placed in front of the eye (4) of the user and spaced apart from the eye (4) of the user and a second electrode (3) configured to provide an electrical reference potential. Also disclosed is a method of examining blinking of an eye (4) of a user, comprising detecting (51) an electrical field in front of the eye (4) of the user and determining (S2) a blinking parameter associated with the eye (4) of the user based on the detecting.

FIG 1

## Description

### Technical Field

The present disclosure generally relates to an eye blink sensor, to a method of examining blinking of an eye of a user and to a computer program.

### Background

Blinking is essential for the healthy functioning of the human eyes. Eye-blinking maintains the integrity of the ocular surface by keeping the eye surface humid. Blinking contributes to drainage of tears, excretion of lipids from Meibomian glands and spreading of lipids for a restoration of the normal structure of tear film and keeps it from drying out. Control of blinking is semi-automatic: blinks are divided on spontaneous, reflex and voluntary. Spontaneous blinks are conducted in the pre-motor brain stem and happen without conscious efforts, like breathing and digestion. Reflex blinks, also known as triggered blinks, occur in response to an external stimulus, which can be tactile, optical or auditory. The role of reflex blinks is to protect eyes from external mechanical irritants. Voluntary blinks are triggered by the central nervous system. The properties of blinks differ, with voluntary blink having the largest impact on the eye, while spontaneous blink is the fastest. Spontaneous blinks might also be incomplete when the upper lid does not cover exposed cornea completely during a blink.

Spontaneous eye blink rate (SEBR) and blink completeness are reduced during demanding cognitive tasks, for example while using visual display terminals. This leads the way to disruption of the balanced delicate irrigation system of the corneal surface and results in its damage.

In recent years, prevalence of electronic device usage in daily life both for professional and leisure tasks increased dramatically, which led to an increase in the occurrence of diseases associated with tear-film deficiency, such as dry eye.

A change in a blinking pattern may be monitored. Based on the monitoring, one may intervene to restore a certain blinking pattern (biofeedback). Blinking patterns can be associated with a number of medical conditions, such as a normal eye, dry eye disease, Tourette syndrome, strokes or disorders of the nervous system, Parkinson's disease, etc. Short term changes in blinking are caused by the activities of the person, for example, a blinking rate is reduced during periods of attention and increased during a conversation and thus monitoring the blinking can be used for the recognition of a human activity. Also state of drowsiness affects the blinking pattern and dynamics of blinking. Increased eyelid closure can indicate episodes of microsleeps, which are important to detect in drivers, pilots and operators of machines for the sake of public safety.

### Summary

There is a need for a sensor and method to monitor the blinking of an individual.

According to a first aspect, an eye blink sensor is provided comprising a detection system configured to detect an electrical field in front of an eye of a user. The electrical field is for example generated by a polarity of the eye. As is known, the retina is mostly negatively charged while the cornea is mostly positively charged, which results in a polarity of the eye and in an electrical field which also extends in front of the eye. In other words, the electrical field in case the eye is open extends into a volume outside the cornea of the eye.

The detection system comprises a first electrode configured to be placed in front of the eye of the user. The first electrode is spaced apart from the eye of the user. The first electrode is for example spaced apart from the cornea of the user, for example by a few mm to a few cm. In one variant, means are provided to apply a predetermined offset potential to the first electrode.

The detection system comprises a second electrode configured to provide an electrical reference potential. The electric reference potential may be a ground potential. The second electrode may be electrically connected to a body of the user and/or grounded. For example, the predetermined offset potential applied to the first electrode results in a predetermined offset voltage between the first electrode and the second electrode. The offset voltage may lie in the range of volts and for example be equal to 2 V. The offset voltage is influenced by the electrical field in front of the eye of the user.

The detection system may comprise at least one third electrode vertically displaced from the first electrode. For example, the detection system comprises a plurality of third electrodes which are each displayed from one another vertically. In one variant, the plurality of electrode is arranged below the first electrode. The first electrode and the at least third electrode are for example not directly electrically coupled to one another such that each of these electrodes may exhibit a different electrical potential.

The first electrode and/or the at least one third electrode is in one example placed on an optically transparent material. The first electrode and/or the at least one third electrode may be disposed on a lens. The lens is for example a lens comprised in glasses. The lens may be an ophthalmic lens. In one variant, the first electrode is placed on a surface configured to face the eye of the user. Also, at least one of the at least one third electrode may be placed on the surface configured to face the eye of the user. The surface may be arranged in front of the cornea of the eye.

The first electrode and/or the at least one third electrode is in one example embedded in an optically transparent material. For example, the first electrode and/or the at least one third electrode is embedded in a lens of glasses. The lens is for example an ophthalmic lens. The first electrode and/or the at least one third electrode may each be embedded in the form of a wire. In other words, an embedded electrode may have the form of a wire. The wire is in one variant dimensioned such that is does not obstruct a user's view.

The detection system may comprise a measurement unit configured to measure at least one electrical parameter between the first electrode and the second electrode. The measurement unit is for example configured to additionally or alternatively measure at least one electrical parameter between the at least one third electrode and the second electrode. The measurement unit is in one variant configured to measure the at least one electrical parameter between the first electrode and the at least one third electrode. For example, the measurement unit is configured to generate a detection signal based on the measurement. The electrical parameter is for example one of an electrical current, an electrical voltage and an electrical impedance.

The eye blink sensor for example further comprises a processing unit configured to determine a blinking parameter associated with the eye of the user. For example, the blinking parameter is determined based on the detected electrical field, for example based on the detection signal.

According to a second aspect, a method of examining blinking of an eye of a user is disclosed. The method comprises detecting an electrical field in front of the eye of the user and determining a blinking parameter associated with the eye of the user based on the detecting.

The blinking parameter of the first aspect and the blinking parameter of the second aspect are the same. The blinking parameter is for example at least one of a blinking frequency, a blinking pattern such as a temporal pattern of voluntary blinks, an inter-blink interval, a blinking speed (a speed of movement of at least one eye lid of an eye of the user), a completeness of blinking, a detection of a voluntary blink, and a detection of a spontaneous blink. In one variant, the blinking parameter is statistics of at least one of a blinking frequency, an inter-blink interval, a blinking pattern such as a temporal pattern of voluntary blinks, a blinking speed (a speed of movement of at least one eye lid of an eye of the user), a completeness of blinking, a detection of a voluntary blink, and a detection of a spontaneous blink.

The step of detecting the electrical field may comprise observing the electrical field for a time period starting with a first point in time and generating a detection signal based on the observing. The step of detecting the electrical field in one example comprises observing the electrical field at different spatial positions for a time period starting with a first point in time and generating detection signals based on the observing. In one variant, the step of detecting the electrical field comprises observing the electrical field at different spatial positions for a time period starting with a first point in time and generating a detection signal for each of the spatial positions based on the observing.

Before the blinking parameter is determined, additional steps may be performed. For example, the detection signal or the detection signals may be adjusted before the blinking parameter is determined. In one variant, an offset is applied to the detection signal(s). For example, the detection signal(s) is/are amplified. Frequency filtering may be applied to the detection signal(s). Peak detection may be performed based on the detection signal(s). In one example, suitable signal processing, for example wavelet processing, is applied to the detection signal(s).

The step of determining the blinking parameter for example comprises comparing the detection signal(s) with at least one reference signal, for example with a plurality of reference signals each of which is associated with a different blinking parameter. The step of determining the blinking parameter may comprise comparing at least two detection signals with at least one reference signal or with one another.

The detection signal is for example compared with at least one reference signal describing a threshold value associated with a voluntary blink or a spontaneous blink. At least two detection signals may be compared with one another to determine a completeness of blinking as the blinking parameter. At least two detection signals may be compared with one another to determine a blinking speed as the blinking parameter. For example, at least two detection signals generated by observing the electrical field at different spatial positions are compared with one another to determine the blinking parameter.

The method for example further comprises determining a correlation between the determined blinking parameter and a predetermined blinking parameter. The predetermined blinking parameter is for example at least one of a predetermined blinking frequency, a predetermined blinking pattern such as a predetermined temporal pattern of voluntary blinks, a predetermined inter-blink interval, a predetermined blinking speed (a predetermined speed of movement of at least one eye lid of an eye of the user), a predetermined completeness of blinking, a predetermined detection of a voluntary blink, and a predetermined detection of a spontaneous blink. In one variant, the predetermined blinking parameter is predetermined statistics of at least one of a blinking frequency, an inter-blink interval, a blinking pattern such as a temporal pattern of voluntary blinks, a blinking speed (a speed of movement of at least one eye lid of an eye of the user), a completeness of blinking, a detection of a voluntary blink, and a detection of a spontaneous blink.

The determined blinking parameter may be compared with predetermined blinking parameters. Based on the comparison, one of the predetermined blinking parameters which has the highest correlation, for example the highest similarity, with the determined blinking parameter may be chosen. The method may also comprise determining an event based on the correlation. For example, an event associated with the chosen predetermined blinking parameter is determined as the event. Such an event may be a detection of tiredness of a user or a detection of an eye of the user being dry or a control command such as a user input.

According to a third aspect, a computer program is provided which, when running on a computer, causes the computer to perform the method steps of the method according to the second aspect. Also provided is a program storage medium on which the program is stored, in particular in a non-transitory form. Also provided is a data stream carrying information which represents the program.

**Brief Description of the Drawings**

Further details, advantages and aspects of the present disclosure will become apparent from the following embodiments taken in conjunction with the drawings, wherein:

Fig. 1 shows an embodiment of an eye blink sensor;
Fig. 2 shows an embodiment of an eye blink sensor;
Fig. 3 shows an amplification circuit;
Fig. 4 shows a method embodiment;
Fig. 5 shows an amplified detection signal and a transformed detection signal;
Fig. 6 shows an amplified detection signal and a transformed detection signal;
Fig. 7 shows an amplified and filtered detection signal and a transformed detection signal; and
Fig. 8 shows detection signals generated by observing an electrical field at different spatial positions.

**Detailed Description**

In the following description, exemplary embodiments of an eye blink sensor and a method of determining blinking of an eye will be explained with reference to the drawings. The same reference numerals will be used to denote the same or similar structural features.

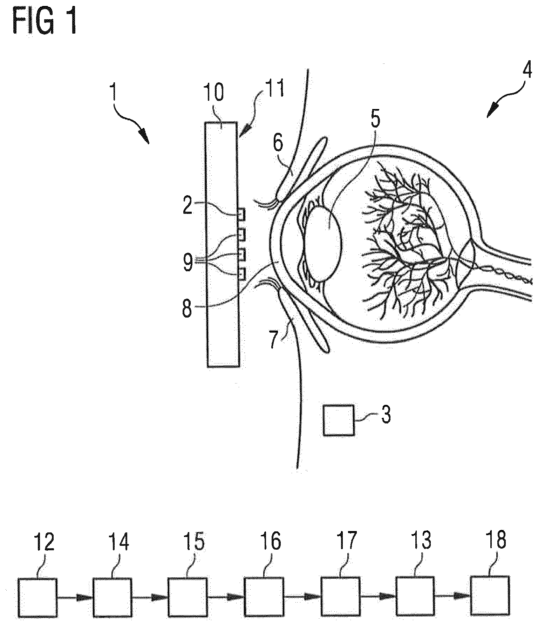

Fig. 1 shows an embodiment of an eye blink sensor 1. The eye blink sensor 1 comprises a detection system. The detection system comprises a first electrode 2 and at a second electrode 3. The detection system is configured to detect an electrical field in front of an eye 4 of a user.

The eye 4 comprises a lens 5. An upper eyelid 6 and a lower eyelid 7 cover a portion of cornea 8 of the eye 4. As is commonly known, and eye 4 of a human exhibits and electrical field. In other words, the eye 4 has a given polarity. The first electrode 2 is configured to be placed in front of the eye 4 and spaced apart from the eye 4. Therefore, the first electrode 2 is influenced by the polarity of the eye 4 at a given position in front of the eye 4. That is, a certain potential can be measured with the first electrode 2 in front of the eye 4.

In difference thereto, the second electrode 3 is configured to provide an electrical reference potential. The second electrode is positioned such that it is not influenced by the polarity of the eye 4. For example, the second electrode 3 is configured to be placed on the skin of the user's head. Therefore, a different potential can be obtained by the first electrode 2 compared to the potential obtained by the second electrode 3. A difference between these potentials is influenced by the polarity of the eye 4. This potential difference is also influenced by a position of the upper eyelid 6 and the lower eyelid 7. In particular, an electric field extending in front of the eye 4 towards the first electrode 2 is changed in case the upper eyelid 6 and/or the lower eyelid 7 are in a closed position in front of the eye 4. That is, a different potential is obtained by the first electrode 2 in case the eye lids 6, 7 are open than in case the eye lids 6, 7 are closed. Since the second electrode 3 is not influenced by an opening or closing of the eye lids 6, 7, a potential difference between the first electrode 2 and the second electrode 3 changes depending on the movement of the eye lid 6 and the movement of the eye lid 7. This change may be observed to determine a blinking state as will be described below.

The eye blink sensor 1 comprises at least one third electrode 9. The third electrode 9 is in the shown embodiment comprised in the detection system. In the shown embodiment, the eye blink sensor 1 comprises three third electrodes 9. The at least one third electrode 9 is configured to be placed in front of the eye 4. The third electrode 9 is (only) vertically displaced from the first electrode 2. In Fig. 1, the three third electrodes 9 are all disposed vertically displaced from and below the first electrode 2. Each of the third electrodes 9 is influenced by the electric field in front of the eye 4. Due to the different relative positions between the eye 4 and the first electrode 2 and between the eye 4 and the third electrode 9, different potentials may be obtained by the second electrode 2 and the third electrode 9. For example, in case the upper eye lid 6 closes until a position below the second electrode 2 but above the third electrode 9, a large potential change can be obtained by the second electrode 2 whilst a lower potential change is obtained by the third electrode 9. In case the upper eye lid 6 closes until a position below the second electrode 2 but above the third electrode 9, a large potential change can be obtained by the second electrode 2 whilst a lower potential change is obtained by the third electrode 9. Differences between these potential changes can for example be used to determine a completeness of blinking, to detect a complete blink or to detect a blinking speed.

The first electrode 2 and the third electrodes 9 are placed on a lens 10 of glasses. The lens is an ophthalmic lens. In the shown embodiment, the first electrode 2 and the third electrodes 9 are placed on a surface 11 of the lens 10 which is configured to face the eye 4. The electrodes are deposited on the surface 11 by vapor deposition. The first electrode 2 and the third electrodes 9 are made of a transparent material such as an inorganic or an organic transparent electrically conducting material. Such an inorganic material is for example a transparent indium tin oxide (ITO), fluorine doped tin oxide (FTO) or doped zinc oxide. Such an organic material is for example based on carbon nanotubes or graphene networks or a polymer such as poly(3,4-ethylenedioxythiophene) or its derivatives.

One or more of the first electrode 2 and the third electrodes 9 may instead be embedded in the lens 10. In this case, the embedded electrode(s) may be formed as a wire embedded into the lens 10. The wire may be made of a metal or a metal alloy and dimensioned so as not to obstruct a view of the user through the lens.

The eye blink sensor 1 comprises a measurement unit 12. The measurement unit 12 is configured to measure an electrical voltage drop between the first electrode 2 and the second electrode 3 and to generate a detection signal based on the voltage drop. It is also configured to measure electrical voltage drops between each of the third electrodes 9 and the second electrode 3 and to generate a detection signal for each of the third electrodes 9 based on the measured voltage drop. Alternatively, a current between the electrodes or an impedance between the electrodes may be measured by the measurement unit 12 in order to generate the detection signal. The detection signal may correspond to a time-dependent measurement. For example, the detection signal describes a time-dependent function of the measured electrical parameter such as a time-dependent function of a voltage drop between two electrodes. The function is in one variant continuous over the time period during which the electrical parameter was measured. This enables an improved signal processing. To generate the detection signal, several measurement values might be interpolated and/or extrapolated.

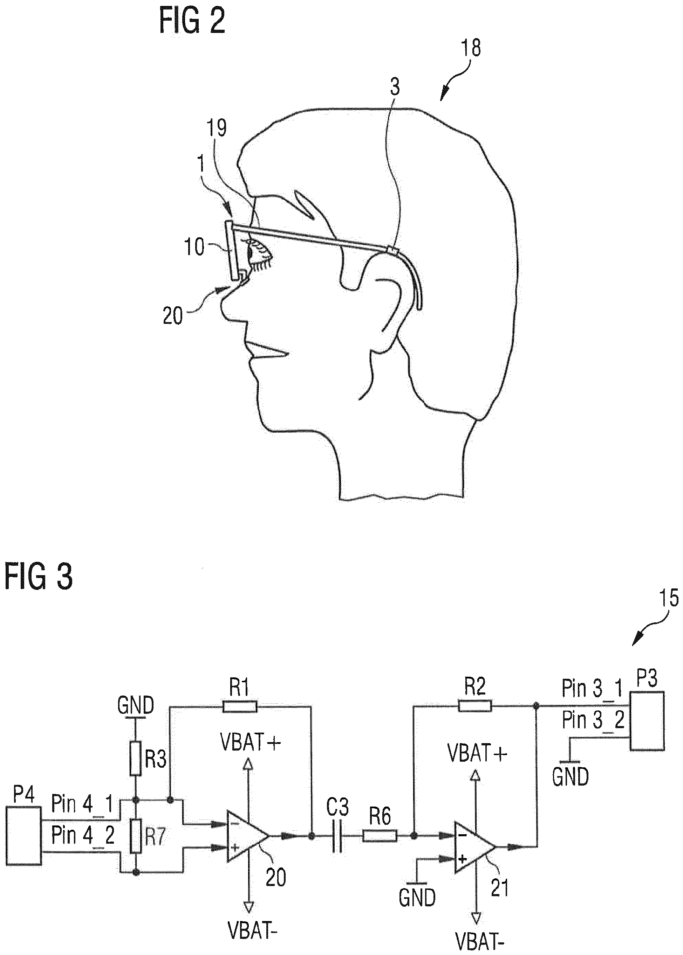

The eye blink sensor 1 further comprises a processing unit 13. The processing unit is configured to determine a blinking parameter associated with the eye 4, based on the detection signal(s). The blinking parameter is one of, or statistics of, a blinking frequency, a blinking pattern such as a temporal pattern of voluntary blinks, an inter-blink interval, a completeness of blinking such as a completeness of 50%, a completeness of 75% and a completeness of 100%, a detection of a voluntary blink, and a detection of a spontaneous blink. Details on how the blinking parameter is determined will be described below with reference to Fig. 3 to Fig. 7.

The eye blink sensor 1 may further comprise an offset unit 14 configured to apply an offset to at least one of the detection signals. The eye blink sensor 1 may further comprise an amplification unit 15 configured to amplify at least one of the detection signals, for example after an offset has been applied. The eye blink sensor 1 may further comprise a filter unit 16 configured to filter frequencies of at least one of the detection signals. It may also comprise a wavelet processing unit 17 configured to apply a wavelet transformation to at least one of the detection signals. Each of the units 14, 15, 16, 17 may be part of the measurement unit 12 or of the processing unit 13 and embodied by hardware or software components. The individual units may adjust the detection signal in a given order, for example the amplification unit 15 adjusts the detection signal, then the filtering unit 16 adjusts the amplified detection signal, then the wavelet processing unit 17 adjusts the amplified and filtered detection signal. Other combinations or sequences of the units 14, 15, 16, 17 are possible. Not all units 14, 15, 16, 17 are required. One or more of the units 14, 15, 16, 17 may be provided multiple times or used multiple times to adjust the detection signal.

The eye blink sensor 1 may also comprise an output unit 18 configured to output a signal based on a determined event as will be described below.

Fig. 2 shows an embodiment of eye blink sensor 1. The eye blink sensor 1 is worn by a user in the same manner as spectacles on the head 18 of the user. The second electrode 3 is arranged on a stem 19 comprised in the eye blink sensor and in contact with the skin of head 18. In the shown configuration this stem 19 corresponds to a spectacle stem and the second electrode 3 is in contact with an ear of the user. Arranged on the stem 19 is lens 10. The lens 10 is comprised in eye blink sensor 1. In the shown embodiment, the lens 10 is supported on the head 18 at a support location 20. The second electrode 3 may be arranged at the support location 20 instead of on the stem 19. In particular, the second electrode 3 may be arranged on a nose pad comprised in the eye blink sensor 1 corresponding to a nose pad of spectacles. The units 12 to 18 may be comprised in a housing arranged on or connected to, for example with a wire, the stem 19.

An embodiment of the amplification unit 15 is shown in Fig. 3. The first electrode 2 is connected to Pin4_1 of terminal P4, whereas the second electrode 3 is connected to Pin4_2. A voltage drop between Pin4_1 and Pin4_2 is input as detection signal. The detection signal is then amplified by amplifier 20. Capacitance C3 blocks current from flowing from Pin4_1 or Pin4_2 to subsequent amplifier 21. The resistors R1, R2, R3, R6 and R7 are dimensioned appropriately to obtain a certain amplification factor. The voltage drop between Pin3_1 and Pin3_2 of terminal P3 corresponds to the amplified detection signal, where Pin3_2 is connected to ground (GND). Of course, other amplification units may be used to amplify the detection signal. For example, R7 has a resistance of 100MOhm, R6 has a resistance of 10kOhm and R2 has a resistance of 20kOhm. Amplifier 20 and amplifier 21 may be amplifiers of the type LMC6082AIM/NOPB of the company Texas Instruments. Resistors R1 and R3 may be omitted. VBAT+ corresponds to a positive supply voltage, whereas VBAT- corresponds to a negative supply voltage.

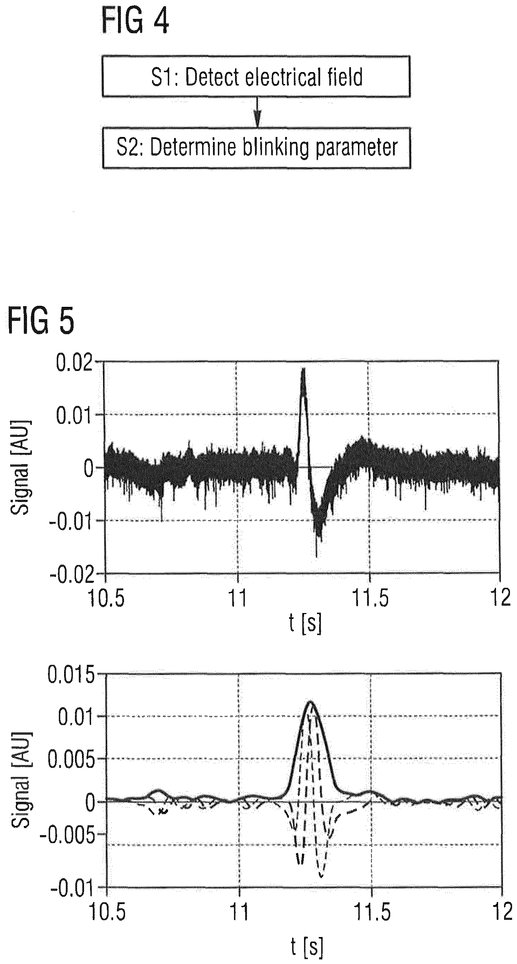

Fig. 4 shows a method embodiment which may be executed by the eye blink sensor 1. The method comprises a first step S1 of detecting the electrical field in front of the eye 4 of the user. For this purpose, the detection system comprised in the eye blink sensor 1 can be used. In a subsequent step S2, the blinking parameter is determined based on the detected electrical field.

The step S2 comprises observing the electrical field for a time period starting with a first point in time and generating a detection signal based on the observing. In particular, the measurement unit 12 is configured to measure the at least one electrical parameter during the time period to generate the detection signal.

In case at least one third electrode 9 is comprised in the eye blink sensor 1, several detection signals may be obtained. In particular, for each third electrode 9, the at least one electrical parameter is measured by measuring unit 12 during the time interval to obtain a detection signal. In other words, the electrical field is observed at different spatial positions to generate the detection signals.

The measurements may be performed for subsequent time periods by measuring unit 12. This results in detection signals generated by observing the electrical field for time periods starting with different points in time. The subsequent time periods do not need to be adjacent to one another. For example, the periods all have a length of 10s and a first time period starts at a first point in time corresponding to 0s. A second time period may start at a second point in time corresponding to 60s. A third time period may start at a point in time corresponding to 1h. That is, the detection signals all describe the electrical field observed during a 10s time interval but at different points in time. A comparison of these detection signals with one another enables the deduction of an event such as the user getting tired, the user getting a dry eye or else. The comparison and deduction may be performed by the processing unit 13.

Before the blinking parameter is determined, the detection signal, the temporal detection signals and/or the spatial detection signals may be adjusted. An offset may be applied. The signal(s) may be amplified or frequency-filtered. Also, wavelet processing may be applied to the signal(s).

Different techniques can be used for detecting of a peak in the measured temporal detection signal. Such techniques comprise a window-threshold method, a wavelet transform, a Hilbert transform, a combined Hilbert and wavelet transform, matching the signal with at least one template, using artificial neural networks, morphological filtering, nonlinear filtering, linear prediction analysis, high order statistics, K-Means clustering, fuzzy C-Means clustering, Empirical Mode Decomposition and hidden Markov models. Such techniques may also use entropy, momentum, a histogram or cumulative distribution function, an intensity weighted variance, a stochastic resonance and/or a smoothed nonlinear energy operator.

One example of wavelet processing is applying a Morlet wavelet transformation to the detection signal, for example to one of the spatial detection signals. The Morlet wavelet describes a wave comprising real and imaginary components which are localized to one another based on a Gaussian envelope. The Morlet wavelet can be described by Formula 1.

$$\psi\left(t\right) = \frac{1}{\sqrt{\pi B}}\, exp^{-\frac{t^2}{B}}\, exp^{j2\pi Ct} \qquad\qquad \text{(Formula 1)}$$

Here, t corresponds to time, B and C are variables and j denotes the imaginary component. The Morlet wavelet transformation is applied by convoluting the detection signal with the real parts of the Morlet wavelet and with the imaginary parts of the Morlet wavelet. This results in a waveform with real and imaginary components. An envelope of this waveform is then obtained by determining a square root of squares of the real component and the imaginary component for each point in time.

The illustration at the top of Fig. 5 shows an amplified spatial detection signal in a time interval of 1.5s starting at a first point in time of 10.5s. The signal amplitude is given in arbitrary units (AU) and the time axis shows time t in seconds. As can be seen, the signal has a peak at a point in time of about 11.25s to a positive value of almost 0.02 AU, then decreases to a negative value lower than -0.01 AU and subsequently returns to essentially 0. The signal includes many high-frequency peaks and valleys with an amplitude range of about 0.002 AU in each direction. That is, a high frequency noise signal is included in the amplified spatial detection signal. These high frequency components may be discarded using the Morlet wavelet transform. As shown in the lower illustration of Fig. 5, an envelope indicated by the solid black line is determined for the real and the imaginary components. The real and imaginary components are indicated underneath the envelope as dashed lines. The real and imaginary components are obtained by convoluting the detection signal with the real and the imaginary parts of the Morlet wavelet function, respectively. The envelope may be referred to as transformed signal and transformed detection signal. The transformed signal no longer includes the high-frequency valleys and peaks and thus contains much less noise. The large signal peak at 11.25s can be clearly seen from the transformed signal. In other words, significant changes in a detection signal may be contained in the transformed detection signal obtained by applying Morlet wavelet processing, whilst irrelevant changes in the detection signal such as noise may be discarded using the Morlet wavelet processing.

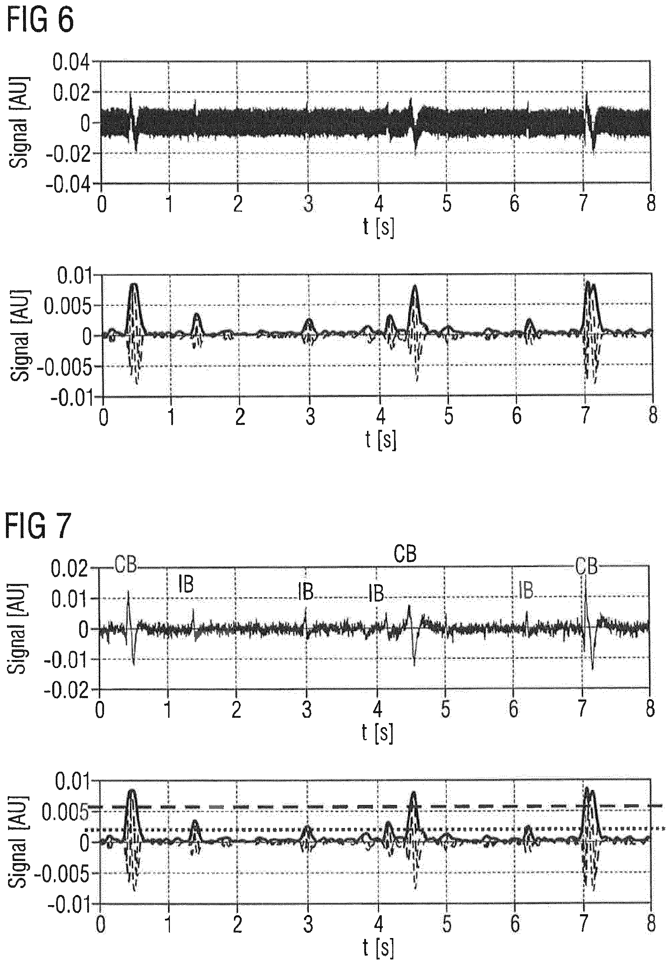

As can be seen in Fig. 6, the detection signal may describe several large peaks and several small peaks. Due to a superimposed noise signal, a detection of these peaks in the detection signal shown in the top illustration of Fig. 6 is not easy. After applying the Morlet wavelet transform, the transformed signal shown in the bottom illustration of Fig. 6 enables a much easier determination of relevant (i.e., significant) large and small peaks. Also in this case, the transformed signal corresponds to the envelope which is obtained as described above and which is indicated by the solid black line.

The top illustration of Fig. 7 shows the same detection signal as the top illustration of Fig. 6, but after frequency filtering. In particular, frequency components around 50Hz have been removed. The resulting filtered detection signal shown in the top illustration shows the individual peaks much more clearly than the unfiltered detection signal of Fig. 6. The signal shown by the black continuous line in the bottom illustration of Fig. 7 corresponds to the envelope obtained as described above and closely matches the envelope shown in the bottom illustration of Fig. 6. This proves that applying the Morlet wavelet transformation is a step that is advantageously performed before detecting individual peaks. In this case, frequency filtering is not required but can also be performed before applying the wavelet transformation.

Different types of blinking have a different effect on the measured electrical field. This can be used to differentiate between an incomplete blink indicated as "IB" in the top illustration of Fig. 7 and a complete blink indicated as "CB" in the top illustration of Fig. 7. The incomplete blink corresponds to a blink in which the eye lids 6, 7 are not fully closed, whereas the complete blink corresponds to a blink in which the eye lids 6, 7 are fully closed. The complete blink may correspond to a voluntary blink. In the bottom illustration of Fig. 7, the horizontal dashed line represents a first reference signal. In this case, the first reference signal corresponds to a first threshold value of around 0.006AU. The horizontal dotted line represents a second reference signal. In this case, the second reference signal corresponds to a second threshold vale of around 0.002AU. In case the transformed signal exceeds the first threshold value, a complete blink "CB" can be determined as the blinking parameter, i.e. the determined blinking parameter indicates a blink at which the eye is 100% closed. In other words, the first threshold value is associated with a complete blink. In case the transformed signal exceeds the second threshold value but does not exceed the first threshold value, an incomplete blink "IB" can be determined as the blinking parameter, i.e. the determined blinking parameter indicates a blink at which the eye is <100% closed, for example 75% closed. In other words, the second threshold value is associated with an incomplete blink. Depending on the thresholds used, other blinking parameters such as a voluntary blink, a spontaneous blink, a triggered blink or else may be determined based on the transformed signal. Also, a blinking frequency or a blinking pattern (i.e., of voluntary blinks) may be determined using the thresholds as described above.

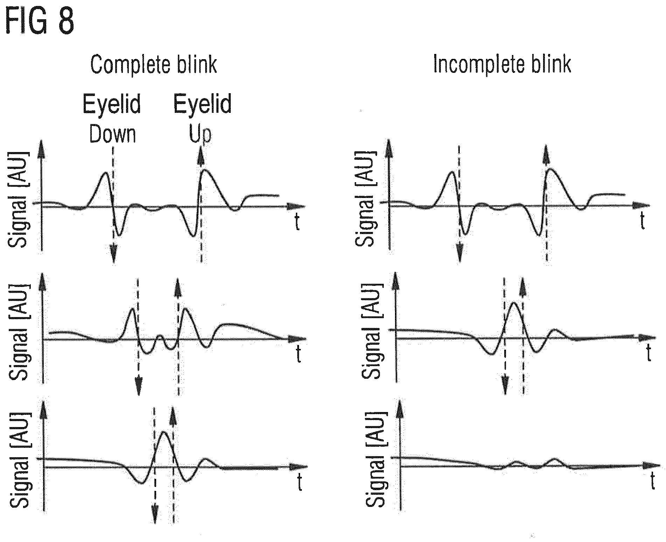

The first and/or second threshold may be a predetermined threshold. It may be adapted using the transformed signal. For example, the user is instructed by output unit 18 to blink voluntarily, for example after a control command is transmitted from the processing unit 13 to the output unit 18. The subsequently detected transformed signal amplitude may be used as a reference for a voluntary blink which means that the first threshold value has to be set lower than this amplitude. Afterwards, spontaneous blinking may be observed which enables setting the first threshold value. Of course, other ways are possible to determine the threshold values, for example using additional sensors which differentiate between different types of blinking. In this example, the detection signal is compared with a reference signal. In addition or alternatively, the signals may be compared with one another as will be described exemplarily below with reference to Fig. 8.

Fig. 8 shows detection signals based on which different blinking parameters can be determined. The illustration at the top corresponds to a transformed detection signal obtained by the first electrode 2. The illustration at the middle corresponds to a transformed detection signal obtained by a third electrode 9 below the first electrode 2. The illustration at the bottom corresponds to a transformed detection signal obtained by a third electrode 9 below the first electrode 2 and the aforementioned third electrode 9. That is, each of the signals reflects the electrical field in front of the eye 4 at the same time but at different spatial positions.

Referring to the illustration on the left side of Fig. 8, the dashed arrows indicate a movement of the upper eyelid 6 in front of the respective electrode into a certain direction. The first peak is measured by the first electrode 2 when the upper eyelid 6 moves into a closing direction. At this point in time, the other two transformed detection signals do not indicate a large potential change. Next, the upper eyelid 6 moves past the third electrode 9 directly below the first electrode 2, resulting in a corresponding peak. Last, the upper eyelid 6 moves past the third electrode 9 at the bottom of the other two electrodes resulting in a peak.

When opening the eye again, the upper eyelid 6 moves in the opposite direction, resulting in peaks in the (i.e., transformed) detection signals of the electrodes when the upper eyelid 6 moves past them. From these detection signals it can be determined that the eye has completely closed since the upper eyelid 6 has passed all electrodes. That is, a completeness of blinking can be determined as being 100%.

In the example shown on the right side in Fig. 8, the eye 4 does not close completely. It can be seen that after passing the upper third electrode 9, the upper eyelid 6 moves upwards again into an opened position. That is, the eyelid 6 does not pass the lower third electrode 9. This can be derived from the transformed detection signal shown on the bottom on the right side of Fig. 8. In this case, a completeness of blinking may be determined as being incomplete or as being, i.e., 66%. In summary, the detection signals are compared with one another to determine a completeness of blinking.

In addition, the speed of an eye lid movement can be determined by comparing the time points at which a peak is detected in the (i.e., transformed) detection signals. This speed may be associated with a blinking parameter, for example with a spontaneous blink resulting in a fast movement of the upper eye lid 6 or a voluntary blink resulting in a slower movement of the upper eye lid 6.

After having determined the blinking parameter, a correlation of the determined blinking parameter and a predetermined blinking parameter may be determined. For example, a blinking frequency (i.e., a frequency of spontaneous blinks) is determined as the blinking parameter. This frequency may be compared with predetermined blinking frequencies which are associated with predetermined events. For example, in case a low blinking frequency is determined and compared with the predetermined blinking frequencies, one of the predetermined frequencies which best matches the determined blinking frequency may be derived as correlating frequency. An event associated with this predetermined frequency can then be determined. The event is for example a state of the eye of the user such as a dry eye or a mood of the user such as tiredness. Based on the event, a signal may be output by signal unit 18, such as a warning signal. A trigger signal may be output which triggers eye blinking. A data message indicating the event may be output by signal unit 18, for example wirelessly transmitted to a base unit.

In one variant, the predetermined blinking parameter is a blinking pattern which is a temporal pattern of voluntary blinks. The blinking pattern may then be compared with a predefined pattern. In case the blinking pattern corresponds to the predefined pattern, an event associated with the predefined pattern can be determined. The event may be used as user input, for example to control an augmented reality device such as a head-mounted display. For example, the user may blink voluntarily three times during two seconds. The predefined pattern may define three voluntary blinks during a maximum time of 2.5 seconds. In this case, the determined blinking pattern of voluntary blinks corresponds to the predefined pattern and the event can be determined. For example, a selection of an object shown to the user on an augmented reality device may be performed as the event. The eye blink sensor 1 may be integrated into the augmented reality device. For example, the augmented reality device is a head-mounted display and the first electrode 2 is arranged on a display portion or a lens of the head-mounted display.

Signals from several electrodes, for example from the first electrode 2 and from a plurality of third electrodes 9, can be combined to increase accuracy of blink detection. The signal corresponding to closing of the eye 4 obtained from an electrode positioned at a lower position of the lens 10 should be preceded by the signal corresponding to closing of the eye 4 obtained from an electrode positioned at an upper position of the lens 10. This is because the upper eyelid 6 first passes the upper position before passing the lower position. The same is true for the opening of the upper eyelid 6: the upper eyelid 6 first passes the lower position before reaching the upper position. Of course, depending on the position of the electrodes with respect to the eye 4, a movement of the lower eyelid 7 may influence the electric field detected by the electrodes. Normally, the lower eyelid 6 is not involved in blinking, but can move due to twitches, spasms or facial gestures and should then be taken into consideration. Also in case of a movement of the lower eyelid 6, electrodes at different vertical positions on the lens 10 yield different detection signals. The blinking parameter can be determined based on the signals of both the electrode at the lower position and the electrode at the upper position.

The blinking parameter can be determined based on at least one peak detected in at least one detection signal obtained with at least one of the second electrode 2 and the third electrode 9. Detecting a peak may include temporal gating. For example, a temporal gating signal is provided defining time intervals during which peaks corresponding to the passage of the eyelid (i.e., blink peaks) can be detected. The temporal gating signal may also define time intervals during which peaks from noise or movement artefacts (i.e., noise peaks) can be detected. The temporal gating signal can be used to detect and/or classify peaks as blink peaks or noise peaks.

In the simplest case a peak is detected and as a result, a binary two-level signal is generated with the state of high indicating detection of a peak and state of low indicating absence of a peak. Alternatively, as a result of peak detection, a digital output indicating a time between the latest two peaks is generated. As a result of peak detection, a confidence signal may be generated indicative of the confidence of the peak detection. For example, the confidence can have a scale of 0 to 1, where 0 indicates a lowest confidence of the peak detection and 1 indicates a maximum confidence. The confidence signal can be modulated onto an output of peak detection to reliably detect blink peaks or noise peaks.

Peaks which are in timing relations associated with eyelid movement may be selected in order to determine the blinking parameter. For example, all peaks are selected which are detected during the time intervals during which peaks corresponding to the passage of the eyelid (i.e., blink peaks) can be detected. The selection may in addition or alternatively be based on a model of eyelid movement in order to find blinks based on the signal(s). In other words, a model of eyelid movement may be used to determine the time intervals of the temporal gating signal described above. The model may be used to validate detected blink peaks.

The eyelid model includes physical and physiological restrictions of eyelid movement, such as limited speed and defined direction. For example, lower eyelid 7 involvement in the blinking process is limited, but can be caused by other factors, such as twitches, spasms or facial gestures. The model can be configured to recognize a twitching of the lower eyelid 7, also called myokymia, and adapt accordingly. Since myokymia can be an interesting physiological signal by itself, the eye blink sensor 1 may be configured to report a recognized myiokymia to the user. The model might assume that peaks cannot be detected with multiple electrodes at the same time.

The model might include a memory of a current state (i.e., a current position) of the upper eyelid 6 and/or the lower eyelid 7. After recognizing an eyelid movement leading to a closed state of the eye 4, the model expects a subsequent upward eyelid movement of upper eyelid 6. In case detected peaks indicate a different eyelid movement, these peaks may be discarded or classified as noise peaks.

The model should be able to adapt to conflicting signals. For example, the model is configured to re-evaluate signals from the past based on new signals.

Peak detection results may be stored in a memory buffer and eyelid movements can then be modelled based on the content of the memory buffer. In one implementation, peak detection results are collected through a complete measurement period and afterwards the eyelid movement model is applied in order to evaluate the signal over the complete measurement period and to derive required metrics such as parameters defining the model. In other words, previously detected blinks, i.e. all previously detected voluntary blinks and all previously detected spontaneous blinks, can be used to adapt the model.

Obviously, the model can be designed to accept peak detection results based on signals from multiple electrodes. The model can also be configured such as to adapt with time to the user. For example, personal timing parameters of the eyelid movement described by the model may be adapted. The model can be adapted by learning from collected signals, for example by collecting distributions of accepted blinking signals and using statistics of the distributions to recognize eyelid movements.

The eyelid model can also provide a feedback signal indicating validated peaks and discarded peaks. In this way peak detection can be optimised to increase the accuracy of peak detection and maximise a ratio of validated peaks and minimise a ratio of discarded peaks. For example, a threshold used for peak detection can be adjusted. In one example, peak detection is started with a low level of the threshold and as a result a lot of false positive peaks are detected. Then, the peaks which match to the eyelid movement are selected based on the model. The feedback signal indicates the peaks which were correctly identified and those which were incorrectly identified. The threshold is then adjusted to maximise specificity and/or sensitivity according to a predefined learning strategy. This can be performed with any other single parameter or with multiple parameters used in peak detection, which depend on the specific detection method. Various techniques can be used to find optimal settings of the peak detection, including machine learning techniques, gradient descend algorithms, deep learning and artificial intelligence.

Optimisation of the parameters can be performed in real time or in a later stage, can be done on-board of the eye blink detector 1 or externally, on the computer or on the cloud computing system and found optimal parameters can then be send back to eye blink detector 1.

A calibration step can be performed for optimization of parameters used for blink detection and the eyelid movement model. For example, the user can be instructed to blink in a specific time interval and/or a specific number of times. This allows adjusting parameters for voluntary blinks. The user can be asked to restrict movements of his body and head 18 and blink freely, which allows to characterise spontaneous blinks, while minimising artefacts.

By knowing, a priori, an expected blinking signal, the model can be fitted to the expected blinking signal in order to more reliably detect a peak. As another implementation, a reference signal can be used for calibrating the model. For example, a photo camera (e.g. of a smartphone) can be directed towards the eye 4 of the user and an image sequence or a video of the eye 4 of the user can be acquired during at least one blink. An analysis of the image sequence or the video can then be performed to detect blinks. This blink detection can be used as a reference to adjust parameters of the model and/or parameters used in peak detection of the eye blink sensor 1.

The eye blink sensor 1 may further include a peak detection unit (not shown) and a modelling unit (not shown), wherein the peak detection unit is configured to detect a peak in at least one of the detection signals and the modelling unit is configured to provide the functionality described above with reference to eyelid movement the model.

The aforementioned method and method steps may be executed by the eye blink sensor 1, in particular by units 12 to 18 comprised in the eye blink sensor 1 and by the peak detection unit and the modelling unit. The method may be performed by a computer. To this end, a computer program may be provided which, when running on the computer, causes the computer to perform the method steps. The program may be stored on a program storage medium or transmitted in the form of a data stream.

**Claims**

1. An eye blink sensor (1) comprising a detection system configured to detect an electrical field in front of an eye (4) of a user, wherein the detection system comprises
a first electrode (2) configured to be placed in front of the eye (4) of the user and spaced apart from the eye (4) of the user and
a second electrode (3) configured to provide an electrical reference potential.

2. The eye blink sensor (1) of claim 1, wherein the detection system comprises at least one third electrode (9) vertically displaced from the first electrode (2).

3. The eye blink sensor (1) of claim 1 or 2, wherein the first electrode (2) and/or the at least one third electrode (9) is placed on an optically transparent material such as a lens (10) of glasses, preferably on a surface (11) configured to face the eye (4) of the user.

4. The eye blink sensor (1) of claim 1 or 2, wherein the first electrode (2) and/or the at least one third electrode (9) is embedded in an optically transparent material such as a lens (10) of glasses, preferably in the form of a wire.

5. The eye blink sensor (1) of any of claims 1 to 4, wherein the detection system comprises a measurement unit (12) configured to measure at least one electrical parameter between the first electrode (2) and the second electrode (3) and/or between the at least one third electrode (9) and the second electrode (3) and to generate a detection signal based on the measurement, wherein the electrical parameter is one of

   a) an electrical current,
   b) an electrical voltage and
   c) an electrical impedance.

6. The eye blink sensor (1) according to any of claims 1 to 5, further comprising a processing unit (14) configured to determine, based on the detected electrical field, preferably based on the detection signal, a blinking parameter associated with the eye (4) of the user.

7. Method of examining blinking of an eye (4) of a user, comprising
detecting (S1) an electrical field in front of the eye (4) of the user; and
determining (S2) a blinking parameter associated with the eye (4) of the user based on the detecting.

8. The eye blink sensor of claim 6 or the method of claim 7, wherein the blinking parameter is at least one of or statistics of:

   a blinking frequency,
   a blinking pattern,
   an inter-blink interval,
   a blinking speed,
   a completeness of blinking,
   a detection of a voluntary blink, and
   a detection of a spontaneous blink.

9. The method of claim 7 or 8, wherein the step of detecting the electrical field comprises at least one of

   observing the electrical field for a time period starting with a first point in time and generating a detection signal based on the observing;
   observing the electrical field at different spatial positions for a time period starting with a first point in time and generating detection signals based on the observing; and

   observing the electrical field at different spatial positions for a time period starting with a first point in time and generating a detection signal for each of the spatial positions based on the observing.

10. The method of claim 9, wherein at least one of the following steps are performed for the detection signal or the detection signals before the blinking parameter is determined:

applying an offset;
amplification;
frequency filtering;
peak detection;
wavelet processing.

11. The method of claim 9 or 10, wherein the step of determining the blinking parameter comprises at least one of
comparing the detection signal with at least one reference signal; and
comparing the detection signals with at least one reference signal or with one another.

12. The method of claim 11, wherein at least one detection signal is compared with at least one reference signal describing a threshold value associated with a voluntary blink or a spontaneous blink.

13. The method of claim 11, wherein at least two detection signals are compared with one another to determine a completeness of blinking or a blinking speed.

14. The method of any of claims 7 to 13, further comprising:

    determining a correlation between the determined blinking parameter and a predetermined blinking parameter; and
    determining an event based on the correlation.

15. A computer program which, when running on a computer, causes the computer to perform the method steps of the method according to any one of claims 7 to 14 and/or a program storage medium on which the program is stored, in particular in a non-transitory form and/or a data stream carrying information which represents the program.

# FIG 1

# FIG 2

# FIG 3

# FIG 4

```
┌─────────────────────────────────┐
│  S1: Detect electrical field    │
└─────────────────────────────────┘
                 │
                 ▼
┌─────────────────────────────────┐
│  S2: Determine blinking parameter│
└─────────────────────────────────┘
```

# FIG 5

FIG 6

FIG 7

# FIG 8

Complete blink                    Incomplete blink

Eyelid    Eyelid
Down      Up

Europäisches Patentamt

European Patent Office

Office européen des brevets

# EUROPEAN SEARCH REPORT

Application Number

EP 19 18 4156

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 101 617 291 B1 (UNIV SEOUL NAT R & DB FOUND [KR]) 18 May 2016 (2016-05-18) * figures 1-2 * * paragraph [0002] - paragraph [0052] * ----- | 1-15 | INV. A61B5/053 A61B5/11 A61B5/00 G02C11/00 A61B3/00 |
| X | LEE JEONG SU ET AL: "Non-contact blink detection glasses utilising transparent conductive film for binary communication", ELECTRONICS LET, IEE STEVENAGE, GB, vol. 51, no. 5, 5 March 2015 (2015-03-05), pages 382-384, XP006051173, ISSN: 0013-5194, DOI: 10.1049/EL.2014.3548 * figures 1-3 * * Introduction, Structure of proposed system, Method * ----- | 1-12,15 | |
| X | WO 2009/128562 A1 (SCALAR CORP [JP]; YAMAMOTO MASAO [JP]) 22 October 2009 (2009-10-22) * figures 1, 7 * * paragraphs [0033], [0075] * ----- | 1,6-9,15 | |

TECHNICAL FIELDS SEARCHED (IPC)

A61B
G02C

The present search report has been drawn up for all claims

| Place of search | Date of completion of the search | Examiner |
|---|---|---|
| The Hague | 8 October 2019 | Knoop, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 18 4156

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2019

| Patent document cited in search report | | Publication date | Patent family member(s) | | Publication date |
|---|---|---|---|---|---|
| KR 101617291 | B1 | 18-05-2016 | NONE | | |
| WO 2009128562 | A1 | 22-10-2009 | JP | 5576268 B2 | 20-08-2014 |
| | | | JP | WO2009128562 A1 | 04-08-2011 |
| | | | WO | 2009128562 A1 | 22-10-2009 |

EPO FORM P0459

For more details about this annex : see Official Journal of the European Patent Office, No. 12/82